(19) Europäisches Patentamt
European Patent Office
Office européen des brevets

(11) Veröffentlichungsnummer: **0 251 077 B1**

(12) **EUROPÄISCHE PATENTSCHRIFT**

(45) Veröffentlichungstag der Patentschrift: **04.12.91**

(51) Int. Cl.[5]: **C07D 277/82**, A61K 31/425

(21) Anmeldenummer: **87108831.6**

(22) Anmeldetag: **19.06.87**

# (54) Neue Tetrahydro-benzothiazole, ihre Herstellung und Verwendung.

(30) Priorität: **21.06.86 DE 3620813**

(43) Veröffentlichungstag der Anmeldung:
**07.01.88 Patentblatt 88/01**

(45) Bekanntmachung des Hinweises auf die Patenterteilung:
**04.12.91 Patentblatt 91/49**

(84) Benannte Vertragsstaaten:
**AT BE CH DE ES FR GB GR IT LI LU NL SE**

(56) Entgegenhaltungen:
**EP-A- 0 186 087**
**DE-A- 1 923 677**
**DE-A- 2 845 857**
**EP-A- 0 207 696**
**DE-A- 2 136 233**
**US-A- 4 337 343**

(73) Patentinhaber: **BOEHRINGER INGELHEIM KG**
**Postfach 200**
**W-6507 Ingelheim am Rhein(DE)**
(84) Benannte Vertragsstaaten:
**BE CH DE ES FR GR IT LI LU NL SE AT**

Patentinhaber: **BOEHRINGER INGELHEIM INTERNATIONAL GmbH**
**Postfach 20**
**W-6507 Ingelheim am Rhein(DE)**
(84) Benannte Vertragsstaaten:
**GB**

(72) Erfinder: **Schneider, Claus, Dr.**
**Albrecht-Dürer-Strasse 19**
**W-6507 Ingelheim am Rhein(DE)**
Erfinder: **Merz, Herbert, Dr.**
**Rotweinstrasse 53**
**W-6507 Ingelheim am Rhein(DE)**
Erfinder: **Sobotta, Rainer, Dr.**
**Ludwig Richter Strasse 6**
**W-6507 Ingelheim am Rhein(DE)**
Erfinder: **Bauer, Rudolf, Dr.**
**Aarstrasse 4**
**W-6200 Wiesbaden(DE)**
Erfinder: **Mierau, Joachim, Dr.**
**An den Weiden 13**
**W-6500 Mainz(DE)**
Erfinder: **Schingnitz, Günter, Dr.**
**Unter den Gärten 18**
**W-6550 Bad Kreuznach 14(DE)**

EP 0 251 077 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Tetrahydro-benzothiazolderivate, ihre Herstellung und ihre Verwendung als Arzneistoffe in üblichen Zubereitungen.

Aus der US-A-4337343 sind Verbindungen bekannt, die strukturelle Verwandschaft mit den erfindungsgemäßen Verbindungen aufweisen. Jedoch enthalten die bekannten Verbindungen am Sechsring eine Aminomethyl- bzw. Alkylaminomethylgruppe, während sich erfindungsgemäß dort eine Amidgruppierung befindet, ferner weisen die bekannten Verbindungen in 2-Position eine Alkylgruppe auf, während erfindungsgemäß dort eine Amino- bzw. substituierte Aminogruppe steht. Zudem werden die Verbindungen nach der US-A insbesondere zur Verbesserung der Durchblutung vorgeschlagen, während die neuen Verbindungen überraschenderweise für die Behandlung der Parkinsonschen Krankheit bzw. des Parkinsonismus geeignet sind. Die aus der DE-A-2845857 bekannten Verbindungen unterscheiden sich ebenfalls in zweierlei Weise von den neuen Verbindungen. Die bekannten Verbindungen sind im Sechsring durch eine Amino- bzw. Alkylaminogruppe, nicht - wie erfindungsgemäß - durch eine Amidgruppierung substituiert, zudem befindet sich in 2-Position nicht eine Amino,- sondern eine Alkylgruppe oder ein H-Atom. Auch diese bekannten Verbindungen sind nicht zur Behandlung der Parkinsonschen Krankheit oder des Parkinsonismus vorgeschlagen worden, sondern für verschiedene andere Zwecke. Durch beide Literaturstellen werden somit die erfindungsgemäßen Verbindungen nicht nahegelegt.

Es wurde gefunden, daß Verbindungen der Formel

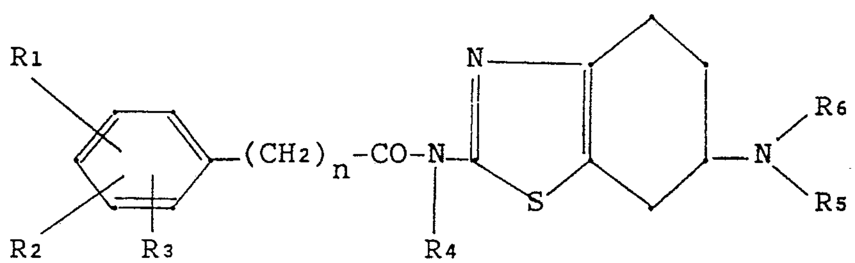

(I),

in der

| | |
|---|---|
| n | für 1, 2 oder 3 steht, |
| $R_1$ | H, F, Cl, Br, $CH_3$, $C_2H_5$, $OCH_3$, $OC_2H_5$, OH, $CF_3$, |
| $R_2$ | H, Cl, $CH_3$, $OCH_3$, $OC_2H_5$, OH, |
| $R_3$ | H, $NH_2$, |
| $R_4$ | H, $CH_3$, $C_2H_5$, |
| $R_5$ und $R_6$ | H, $C_1$-$C_4$-Alkyl, Allyl, |

bedeuten, und ihre Säureadditionssalze als Arzneistoffe verwendbar sind.

Im Rahmen der obigen Definitionen können $R_1$ bis $R_6$ gleich oder verschieden sein und verzweigte oder unverzweigte Kohlenwasserstoffreste enthalten bzw. darstellen.

Bevorzugt steht $R_1$ für H, $OCH_3$, OH, Cl, Br, $CH_3$ oder $C_2H_5$, $R_2$ für H, $OCH_3$ oder Cl, $R_4$ für H oder $CH_3$, $R_5$ für H, $C_1$-$C_3$-Alkyl oder Allyl, $R_6$ für H, $C_1$-$C_3$-Alkyl oder Allyl.

Besonders hervorzuheben sind diejenigen Verbindungen, in denen $R_1$ $OCH_3$ oder OH, $R_2$, $R_3$ und $R_4$ H, $R_5$ und $R_6$ H oder $C_1$-$C_3$-Alkyl bedeuten.

Der Index n steht bevorzugt für 2 oder 3, vor allem für 2.

Falls mindestens einer der Reste $R_1$ bis $R_4$ eine andere Bedeutung als Wasserstoff hat, so ist bevorzugt die 4-Stellung substituiert, während ein gegebenenfalls vorhandener weiterer Substituent sich vorzugsweise in 3-Stellung befindet.

Typische Rest der Formel

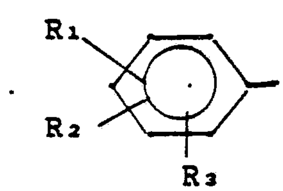

(II)

sind z.B. 4-Methoxyphenyl, 4-Chlorphenyl, 4-Hydroxyphenyl, 3,4-Dimethoxyphenyl, 4-Hydroxy-3-methoxyphenyl, 4-Methylphenyl, 3,5-Dichlor-4-aminophenyl.

Die neuen Verbindungen können als Racemate oder reine Enantiomere, aber auch als Gemische der Enantiomeren in beliebigen Verhältnissen vorliegen. Im allgemeinen ist eines der Enantiomeren eines Racemats stärker wirksam als das andere.

Die Herstellung der neuen Verbindungen kann nach an sich bekannten Methoden erfolgen:

1. Reduktive Aminierung einer Verbindung der Formel

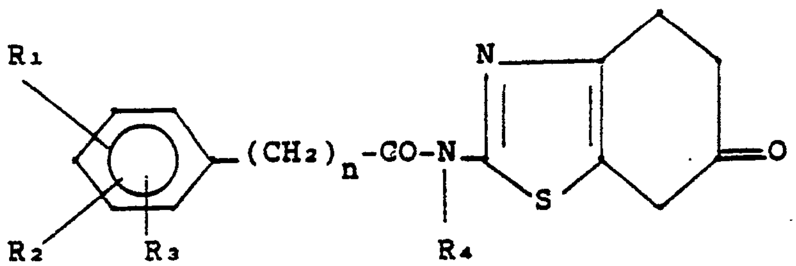

(III),

in der n und $R_1$ bis $R_4$ die oben angegebene Bedeutung haben, mit einer Verbindung der Formel

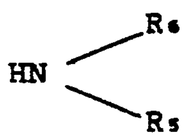

(IV),

in der $R_5$ und $R_6$ die obige Bedeutung haben und einem Reduktionsmittel.

Als Reduktionsmittel können Wasserstoff und Hydrierungskatalysatoren, z.B. Raney-Nickel, Platin, Palladium, oder komplexe Hydride, z.B. Natriumborhydrid, verwendet werden. Als Reaktionsmedium eignen sich unter den Reaktionsbedingungen inerte polare organische Lösungsmittel, z.B. niedere aliphatische Alkohole wie Methanol, Ethanol. Die Umsetzung erfolgt bevorzugt unter leichter Kühlung (z.B. wenn $NaBH_4$ als Reduktionsmittel verwendet wird), bei Verwendung von Wasserstoff/Katalysator gegebenenfalls unter Erwärmen und unter Druck.

2. Zur Herstellung von Verbindungen der Formel I, die eine phenolische OH-Gruppe enthalten, kann auch ein entsprechender Ether, z.B. der Methylether, einer üblichen Etherspaltung, beispielsweise mit Borbromid, unterworfen werden. Als Lösungsmittel eignen sich z.B. halogenierte Kohlenwasserstoffe, etwa Methylenchlorid oder Ethylenchlorid. Die Umsetzung wird zweckmäßig bei Raumtemperatur ausgeführt.

3. Zur Herstellung von Verbindungen der Formel I, in denen die beiden Reste $R_5$ und $R_6$ nicht Wasserstoff bedeuten, kann eine entsprechende Verbindung, in der $R_6$ Wasserstoff ist, mit einer Verbindung der Formel

$x - R'_6$ (V)

umgesetzt werden, in der $R'_6$ dieselbe Bedeutung wie $R_6$ hat, ausgenommen Wasserstoff, und x eine unter Einfführung von $R'_6$ in die Aminogruppe abspaltbare Gruppe darstellt, z.B. ein Halogenatom.

Soweit die Ausgangsstoffe nicht bekannt sind, können sie nach üblichen Methoden hergestellt werden.

Verbindungen der Formel III beispielsweise lassen sich durch Umsetzung von Säurechloriden der Formel

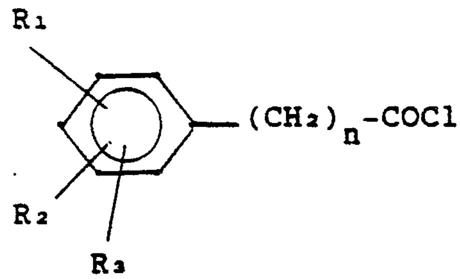

(VI)

worin n, $R_1$, $R_2$ und $R_3$ die obige Bedeutung haben, mit 6-Oxo-2-aminotetrahydrobenzothiazol in der Wärme, vorzugsweise in Gegenwart eines tertiären aliphatischen Amins wie Triethylamin, in einem inerten organsichen Lösungsmittel, herstellen.

Endprodukte der Formel I, die zunächst als Basen erhalten werden, können in üblicher Weise in Säureadditionssalze, zunächst erhaltene Säureadditionssalze in Basen oder Salze anderer Säuren umgewandelt werden.

Als Säuren eignen sich alle anorganischen oder organischen Säuren, die mit den erfindungsgemäßen Basen hinreichend stabile Salze ergeben.

Für die unmittelbare Anwendung als Arzneistoff dienen die Salze physiologisch gut verträglicher Säuren, z.B. die Hydrochloride, Hydrobromide, Sulfate, Methansulfonate, Succinate, Fumarate, Maleinate, Citrate, Formiate.

Die erfindungsgemäßen Verbindungen enthalten ein chirales Zentrum und fallen daher im allgemeinen als Racemate an, die dann gewünschtenfalls mit üblichen optisch aktiven Säuren in die Enantiomeren aufgetrennt werden können, z.B. mit Weinsäure, O,O-Dibenzoylweinsäure, Camphersulfonsäure, α-Methoxyphenylessigsäure.

Wird, z.B. in Verfahren 2 oder 3, ein optisch aktives Ausgagsprodukt eingesetzt, können die Enantiomeren auch unmittelbar erhalten werden.

Die neuen Verbindungen sind als Arzneistoffe, insbesondere zur Behandlung der Parkinsonschen Krankheit bzw. des Parkinsonismus geeignet. Sie können ferner zur Prolactinhemmung und zur Behandlung der Schizophrenie verwendet werden.

Die erfindungsgemäßen Verbindungen zeigen ein besonders günstiges Wirkungsprofil. Hervorzuheben ist, daß

- die Wirkung lange anhält (bis ca. 20 Stunden),
- im therapeutischen Dosisbereich keine Emesis auftritt und
- keine adrenerge Wirkung zu beobachten ist.

Verbindungen mit einem derartigen Wirkungsprofil sind bisher nicht beschrieben worden.

Der Wirkungsnachweis kann am Affen erfolgen (MPTP-Modell).

Bestimmung der Anti-Parkinsonismus- bzw. der Anti-Parkinson-Wirkung

Die Entdeckung des Neurotoxins 1-Methyl-4-phenyl-1,2,3,6-tetrahydro-pyridin (MPTP) (Langston et al., Science 219, 979 (1983)) hat ein Tiermodell für die Parkinsonsche Erkrankung zur Verfügung gestellt.

Das durch MPTP beim Menschen und beim Affen ausgelöste, irreversible, neurologische Krankheitsbild ähnelt in seiner klinischen, pathologischen, biochemischen und pharmakologischen Ausprägung weitgehend der idiopathischen Parkinsonschen Erkrankung (Markey et al., Nature 311, 464 (1984)). Ursache dieser Übereinstimmung ist, daß MPTP selektiv jene kleine Gruppe dopaminerger Nervenzellen in der Substantia nigra des Gehirns zerstört, welche auch bei der natürlich auftretenden Parkinsonschen Erkrankung durch degenerative Prozesse zerstört werden. Es wird auch diskutiert, ob auch die Ursache der idiopathischen Parkinsonschen Erkrankung im Organismus entstehende MPTP oder eine ähnliche chemische Verbindung sei ( Snyder, S.H., Nature 311, 514 (1984)). Möglicherweise bedingt durch den spezifischen Metabolismus des MPTP ist die klinische Ausprägung des MPTP-Parkinsonbildes bisher außer beim Menschen nur beim Affen nachweisbar.

Das an Rhesusaffen verwirklichte MPTP-Modell ist daher in hervorragendem Maße geeignet, die Wirkung von Anti-Parkinson-Medikamenten zu prüfen. Rhesusaffen wurde MPTP (3 Tage lang 1 × 0,15 mg/kg i.m. täglich 3 Tage Pause, 3 Tage lang 1 × 0,30-0,40 mg/kg täglich) appliziert; sie wiesen folgende Symptome auf: die Tiere waren akinetisch und nicht in der Lage, Wasser und Futter aufzunehmen. Sie zeigten eine typische gebeugte Haltung; gelegentlich traten kataleptische Zustände auf. Die Extremitäten

wiesen einen Rigor auf, welcher bei passiver Bewegung von klonischen Krämpfen durchbrochen wurde. Willkürbewegungen des Rumpfes und der Extremitäten waren in der Regel durch stärkste, schmerzhafte Reize nicht auszulösen.

Nach der intramuskulären Gabe erfindungsgemäßer Verbindung treten im zeitlichen Abstand von einigen Minuten erste Willkürbewegungen auf, die von einer allmählichen, weitgehenden Normalisierung der Motorik gefolgt sind. Die Tiere sind dann in der Lage, Nahrung aufzunehmen. Sie erhalten sich innerhalb ihrer Käfige regelrecht, dies gilt auch hinsichtlich Vigilanz und artspezifischen Verhaltens. Als Restsymptomik wird gelegentlich vorübergehender, leichter Ruhetremor und Verringerung der groben Kraft registriert.

Die Wirkung der Verbindungen läßt z.T. erst nach etwa 20 Stunden nach, und die Tiere verfallen wieder in die oben beschriebene Parkinson-Symptomatik; eine erneute Applikation der Verbindung führt wieder zur Besserung bzw. weitgehender Aufhebung der klinisch pathologischen Erscheinung. Die vorteilhafte Wirkung der Verbindungen laßt sich somit reproduzieren.

Zur therapeutischen Anwendung der neuen Verbindungen werden übliche galenische Zubereitungen hergestellt, z.B. Tabletten, Dragées, Suppositorien, Pulver, Suspensionen, Lösungen.
Die Dosis pro Tag beträgt 0,1 bis 10 mg/kg, vorzugsweise 0,5 bis 5 mg/kg Körpergewicht; sie wird in einer oder mehreren Einzeldosen verabreicht.

Beispiele für Arzneimittelzubereitungen gemäß der Erfindung (Angabe in Gewichtsteilen):

Dragées

| | |
|---|---|
| 5,0 Teile | Wirkstoff gemäß der Erfindung |
| 33,5 Teile | Milchzucker |
| 10,0 Teile | Maisstärke |
| 1,0 Teile | Gelatine |
| 0,5 Teile | Magnesiumstearat |

Die pulverisierten Bestandteile Wirkstoff, Milchzucker und Maisstärke werden mit wäßriger Gelatinelösung granuliert und getrocknet. Das Granulat wird mit dem Magnesiumstearat vermischt und zu Dragéekernen von 50 mg Gewicht verpreßt und nach bekannten Methoden überzogen.

Suppositorien

| | |
|---|---|
| 10 Teile | Wirkstoff gemäß der Erfindung |
| 1690 Teile | Zäpfchenmasse (z.B Witepsol W 45) |

Die feingepulverte Substanz wird mittels eines Homogenisators in der geschmolzenen, auf 40° C abgekühlten Zäpfchenmasse gleichmäßig verteilt. Aus der Mischung werden Zäpfchen mit einem Gewicht von 1,7 g geformt.

Die nachstehenden Beispiele sollen die Erfindung näher erläutern:

Beispiel 1

2-(4-Methoxyphenylpropionyl)amino-6-n-propylamino-4,5,6,7-tetrahydrobenzothiazol

a) Herstellung des Racemats

15,1 g (0,09 Mol) 6-Oxo-2-amino-tetrahydrobenzothiazol und 20,5 g (0,1 Mol) 4-Methoxyphenylpropionsäurechlorid werden in 450 ml Tetrahydrofuran und 0,1 Mol Triethylamin 2 Stunden unter Rückfluß zum Sieden erhitzt, anschließend auf Eis gegossen und mit Essigester extrahiert. Nach dem Trocknen kristallisiert beim Einengen 2-(4-Methoxyphenylpropionyl)amino-6-oxo-tetrahydrobenzothiazol (17,5) aus, das ohne weitere Reinigung, in Methanol gelöst, mit Propylamin im Autoklav reduktiv aminiert wird (Raney-Nickel, 5 bar, 60° C). Nach dem Absaugen des Katalysators wird das Lösungsmittel abdestilliert. Der Rückstand kristallisiert aus i-Propylether.

Ausbeute: 12,5 g (63 % d.Th.)
Base: Fp. 105-106° C (aus Essigester umkristallisiert)
Dihydrochlorid: Fp. 259-261° C.

b) Trennung des Racemats

Zu einer Suspension des nach a) erhaltenen Produkts (9,3 g, 0,025 Mol) in 200 ml Wasser werden 3,75

g (0,025 Mol) L-(+)-Weinsäure [Aldrich: [$\alpha_D^{20}$ ]+ 12° (c = 20 $H_2O$)] gegeben. Die Mischung wird 15 Minuten am Rückfluß zum Sieden erhitzt und filtriert. Die nach einem Tag ausgefallenen farblosen Kristalle werden abgesaugt. Dieses L-(+)-weinsaure Salz wird fünfmal aus 75 ml Wasser umkristallisiert. Durch weiteres Umkristallisieren ändert sich der Drehwert $[\alpha]_D^{20}$ - 45,5° (c =1, $CH_3OH$) der freigesetzten Base nicht mehr.

Aus dem reinen L-(+)-weinsauren Salz wird mit konz. Ammoniak die Base freigestetzt und mit Essigester extrahiert. Nach dem Waschen und Trocknen (Magnesiumsulfat) wird das Lösungsmittel im Vakuum entfernt: Durch Behandeln mit etherischer Salzsäure kristallisiert das Dihydrochlorid des (-)-Enantiomeren.

Ausbeute: 0,9 g, Fp. 261 - 262° C
$[\alpha]_D^{20}$ - 41,1° (c =1, $CH_3OH$)

Beispiel 2

2-(4-Hydroxyphenylpropionyl)amino-6-n-propylamino-4,5,6,7-tetrahydrobenzothiazol

9,6 g (0,026 Mol) der nach Beispiel 1 erhaltenen Verbindung werden in 300 ml Methylenchlorid gelöst und mit 90 ml Bortribromid bei 15° C 3 Stunden gerührt. Das Reaktionsgemisch wird mit Wasser versetzt und mit konz. Ammoniak alkalisch gemacht. Die organische Phase wird mit Methylenchlorid extrahiert, getrocknet und eingeengt. Aus dem Rückstand wird mit ethanolischem Bromwasserstoff das Dihydrobromid der Titelverbindung erhalten.

Ausbeute: 4,95 g (49% d. Th.)
Fp. 228-229° C.

Weitere Beispiele

| Nr. | $R_1$ | $R_2$ | $R_3$ | $R_4$ | $R_5$ | $R_6$ | n | Fp.[°C] |
|---|---|---|---|---|---|---|---|---|
| 3 | 4-$OCH_3$ | H | H | H | $C_2H_5$ | H | 2 | 224-225 (Fumarat) |
| 4 | 4-$OCH_3$ | H | H | H | $CH_3$ | H | 2 | 202-204 (Fumarat) |
| 5 | 4-OH | H | H | H | $C_2H_5$ | H | 2 | 164-165 (Dihydro-bromid) |
| 6 | 4-OH | H | H | H | $CH_3$ | H | 2 | 239-240 (Base) |
| 7 | 4-$CH_3$ | H | H | H | n-$C_3H_7$ | H | 2 | >260 (Fumarat) |
| 8 | 2-$OCH_3$ | H | H | H | n-$C_3H_7$ | H | 2 | 216-217 (Oxalat) |
| 9 | 3-$OCH_3$ | H | H | H | n-$C_3H_7$ | H | 2 | |
| 10 | 4-$OCH_3$ | H | H | $CH_3$ | n-$C_3H_7$ | H | 2 | |
| 11 | 4-$OCH_3$ | H | H | H | n-$C_3H_7$ | $C_6H_5$<br>\|<br>$C_2H_5$ | 2 | |
| 12 | 4-$OCH_3$ | H | H | H | n-$C_3H_7$ | n-$C_3H_7$ | 2 | |
| 13 | 4-Cl | H | H | H | n-$C_3H_7$ | H | 2 | |
| 14 | 3-Cl | 4-Cl | H | H | n-$C_3H_7$ | H | 2 | |
| 15 | H | H | H | H | n-$C_3H_7$ | H | 2 | >260 (Fumarat) |
| 16 | 4-OH | H | H | H | $CH_3$ | $CH_3$ | 2 | 259-260 Dihydro-bromid) |
| 17 | 4-$CF_3$ | H | H | H | n-$C_3H_7$ | H | 2 | |

| Nr. | $R_1$ | $R_2$ | $R_3$ | $R_4$ | $R_5$ | $R_6$ | n | Fp.[°C] |
|---|---|---|---|---|---|---|---|---|
| 18 | 4-$OCH_3$ | H | H | H | $CH_3$ | $CH_3$ | 2 | > 260 (Mono-hydro-chlorid) |
| 19 | 4-$OCH_3$ | H | H | H | H | H | 2 | 115-117 (Base) |
| 20 | 4-$OCH_3$ | 3-$OCH_3$ | H | H | n-$C_3H_7$ | H | 2 | |
| 21 | 4-$C_2H_5$ | H | H | $C_2H_5$ | $CH_2$=$CH$-$CH_2$ | $CH_2$=$CH$-$CH_2$ | 2 | |
| 22 | 3-Cl | 5-Cl | 4-$NH_2$ | H | n-$C_3H_7$ | H | 2 | |
| 23 | 4-$CF_3$ | H | H | H | $CH_3$ | $C_2H_5$ | 2 | |
| 24 | 2-F | 4-$OCH_3$ | H | H | i-$C_3H_7$ | H | 2 | |
| 25 | 4-OH | 2-$CH_3$ | H | H | n-$C_4H_9$ | H | 1 | |
| 26 | 4-$OCH_3$ | H | H | H | n-$C_3H_7$ | H | 3 | 93-94 Dihydro-chlorid |
| 27 | 4-Br | H | H | $CH_3$ | t-$C_4H_9$ | H | 2 | |
| 28 | 3-OH | H | H | H | i-$C_3H_7$ | H | 3 | |
| 29 | 4-$OCH_3$ | H | H | H | n-$C_3H_7$ | n-$C_3H_7$ | 3 | |
| 30 | 4-$OC_2H_5$ | H | H | H | n-$C_4H_9$ | H | 2 | |
| 31 | 4-$C_2H_5$ | H | H | H | n-$C_4H_9$ | H | 2 | |
| 32 | 4-$C_2H_5$ | H | H | H | i-$C_4H_9$ | H | 3 | |

| Nr. | $R_1$ | $R_2$ | $R_3$ | $R_4$ | $R_5$ | $R_6$ | n | Fp.[°C] |
|---|---|---|---|---|---|---|---|---|
| 33 | 3-$OC_2H_5$ | 4-$OC_2H_5$ | H | H | n-$C_4H_9$ | H | 3 | |
| 34 | 3-OH | 5-OH | H | H | $C_2H_5$ | $CH_3$ | 2 | |
| 35 | 4-$OCH_3$ | H | H | H | n-$C_4H_9$ | n-$C_4H_9$ | 2 | |
| 36 | n-$OCH_3$ | H | H | H | n-$C_3H_7$ | H | 1 | 167-168 Difumarat |
| 37 | 3-$OCH_3$ | 4-$OCH_3$ | H | H | $C_2H_5$ | $C_2H_5$ | 3 | |

## Patentansprüche

**1.** Tetrahydro-benzothiazole der Formel

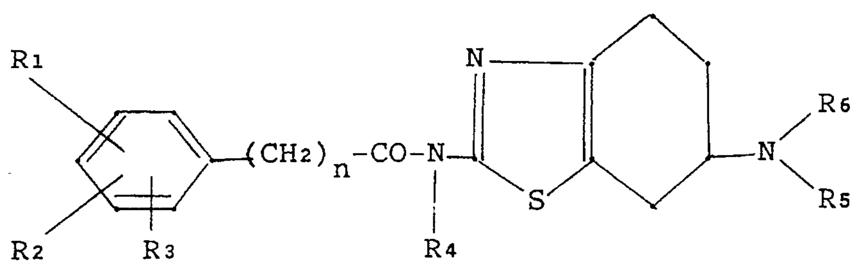

(I),

in der

| | |
|---|---|
| n | für 1, 2 oder 3 steht, |
| $R_1$ | H, F, Cl, Br, $CH_3$, $C_2H_5$, $OCH_3$, $OC_2H_5$, OH, $CF_3$ |
| $R_2$ | H, Cl, $CH_3$, $OCH_3$, $OC_2H_5$, OH, |
| $R_3$ | H, $NH_2$, |
| $R_4$ | H, $CH_3$, $C_2H_5$, |
| $R_5$ und $R_6$ | H, $C_1$-$C_4$-Alkyl, Allyl, |

bedeuten, und ihre Säureadditionssalze.

**2.** Verbindungen nach Anspruch 1, worin

| | |
|---|---|
| $R_1$ | für H, $OCH_3$, OH, Cl, Br, $CH_3$ oder $C_2H_5$, |
| $R_2$ | für H, $OCH_3$ oder Cl, |
| $R_3$ | für H oder $NH_2$, |
| $R_4$ | für H oder $CH_3$, |
| $R_5$ | für H, $C_1$-$C_3$-Alkyl oder Allyl, |
| $R_6$ | für H, $C_1$-$C_3$-Alkyl oder Allyl steht und |
| n | die obige Bedeutung hat. |

**3.** Verbindungen nach Anspruch 1, worin $R_1$ für $OCH_3$ oder OH, $R_2$, $R_3$ und $R_4$ für H, $R_5$ und $R_6$ für H oder $C_1$-$C_3$-Alkyl stehen und n die obige Bedeutung hat.

**4.** Verbindungen nach einem der vorhergehenden Ansprüche, worin n 2 oder 3 bedeutet.

5. Verbindungen nach Anspruch 1, 2 oder 3, worin n 2 bedeutet.

6. 2-(4-Methoxyphenylpropionyl)amino-6-n-propylamino-4,5,6,7-tetrahydrobenzothiazol als Racemat oder in Form des wirksamen Enentiomeren, jeweils als freie Base oder in Form der Säureadditionssalze.

7. Arzneimittel, gekennzeichnet durch einen Gehalt an einer Verbindung nach einem der Ansprüche 1 bis 6, neben üblichen Hilfs- und/oder Trägerstoffen.

8. Verwendung von Verbindungen nach einem der Ansprüche 1 bis 6 bei der Herstellung von Arzneimitteln für die Behandlung der Parkinsonschen Krankheit bzw. des Parkinsonismus, für die Behandlung der Schizophrenie und für die Prolactinhemmung.

9. Verfahren zur Herstellung der Verbindungen nach Anspruch 1 bis 6 nach an sich bekannten Methoden, dadurch gekennzeichnet, daß man

a) eine Verbindung der Formel

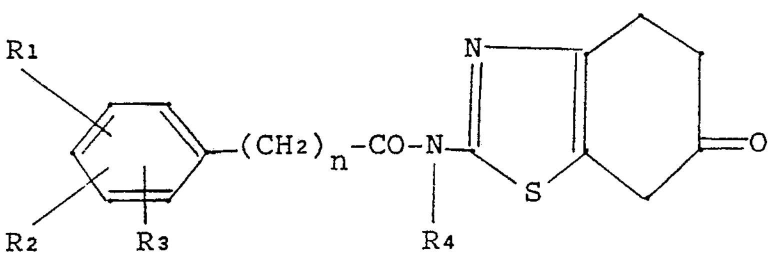

(III)

mit einer Verbindung der Formel

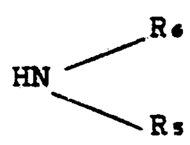

(IV)

unter den Bedingungen der reduktiven Aminierung umsetzt

oder daß man

b) zur Herstellung einer Verbindung der Formel I mit phenolischer OH-Gruppe eine entsprechende Verbindung mit einer Ethergruppe einer Etherspaltung unterwirft

oder daß man

c) zur Herstellung von Verbindungen der Formel I, in denen die beiden Reste $R_5$ und $R_6$ nicht Wasserstoff bedeuten, einen Verbindung der Formel I mit $R_6$ gleich Wasserstoff mit einer zur Einführung des Restes $R'_6$ ($R'_6$ gleich $R_6$, ausgenommen Wasserstoff) geeigneten Verbindung

x - $R'_6$ (V)

umsetzt, worin $R'_6$ die oben angegebene Bedeutung hat und x eine unter Einführung der Gruppe $R'_6$ in die Aminogruppe abspaltbare Gruppe bedeutet,

und daß man gewünschtenfalls die erhaltenen Racemate in die Enantiomeren auftrennt und/oder erhaltene Basen in Säureadditionssalze, erhaltene Säureadditionssalze in die freien Basen oder in Salze anderer Säuren überführt.

**Claims**

**1.** Tetrahydro-benzothiazoles of the formula

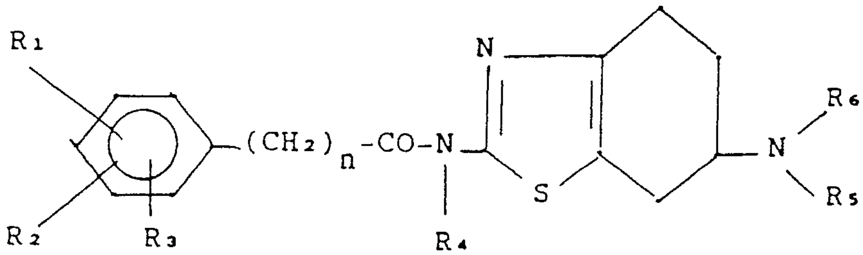

(I)

in which

| | |
|---|---|
| n | represents 1, 2 or 3, |
| $R_1$ | denotes H, F, Cl, Br, $CH_3$, $C_2H_5$, $OCH_3$, $OC_2H_5$, OH or $CF_3$, |
| $R_2$ | denotes H, Cl, $CH_3$, $OCH_3$, $OC_2H_5$ or OH, |
| $R_3$ | denotes H or $NH_2$, |
| $R_4$ | denotes H, $CH_3$ or $C_2H_5$, and |
| $R_5$ and $R_6$ | denotes H, $C_{1-4}$-alkyl or allyl, |

and the acid addition salts thereof.

**2.** Compounds according to claim 1, in which

| | |
|---|---|
| $R_1$ | represents H, $OCH_3$, OH, Cl, Br, $CH_3$ or $C_2H_5$, |
| $R_2$ | represents H, $OCH_3$ or Cl, |
| $R_3$ | represents H or $NH_2$, |
| $R_4$ | represents H or $CH_3$, |
| $R_5$ | represents H, $C_{1-3}$-alkyl or allyl, |
| $R_6$ | represents H, $C_{1-3}$-alkyl or allyl and |
| n | has the above meaning. |

**3.** Compounds according to claim 1, in which
$R_1$ represents $OCH_3$ or OH,
$R_2$, $R_3$ and $R_4$ represent H, and
$R_5$ and $R_6$ represent H or $C_{1-3}$-alkyl, and n has the above meaning.

**4.** Compounds according to one of the previous claims, in which n denotes 2 or 3.

**5.** Compounds according to claim 1, 2 or 3, in which n denotes 2.

**6.** 2-(4-Methoxyphenylpropionyl)amino-6-n-propylamino-4,5,6,7-tetrahydrobenzothiazole as a racemate or in the form of the active enantiomer, in each case as the free base or in the form of the acid-addition salts.

**7.** Medicaments, characterised in that they contain a compound according to one of claims 1 to 6, together with conventional auxiliaries and/or excipients.

**8.** Use of compounds according to one of claims 1 to 6 in the preparation of drugs for the treatment of Parkinson's disease or Parkinsonism, for the treatment of schizophrenia and for prolactine inhibition.

**9.** Process for the preparation of the compounds according to claim 1 to 6 by methods which are known per se, characterised in that

a) a compound of the formula

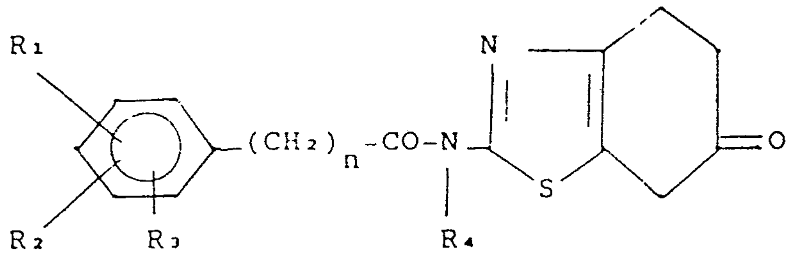

(III)

is reacted with a compound of the formula

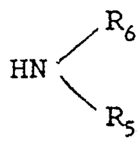

(IV)

under the conditions of reductive amination,

or in that

b) for the preparation of a compound of formula I having a phenolic OH group, an appropriate compound having an ether group is subjected to ether cleavage,

or in that

c) for the preparation of compounds of formula I in which neither radical $R_5$ or $R_6$ denotes hydrogen, a compound of the formula I where $R_6$ equals hydrogen is reacted with a compound

x - $R'_6$ (V)

which is suitable for the introduction of the radical $R'_6$ ($R'_6$ equals $R_6$, apart from hydrogen), and in which $R'_6$ has the above-mentioned meaning and x denotes a group which can be cleaved on introduction of the group $R'_6$ into the amino group,

and in that the racemates obtained are resolved, if desired, into the enantiomers, and/or bases obtained are converted into acid-addition salts, and acid-addition salts are converted into the free bases or into salts of other acids.

**Revendications**

**1.** Tétrahydrobenzothiazoles de formule

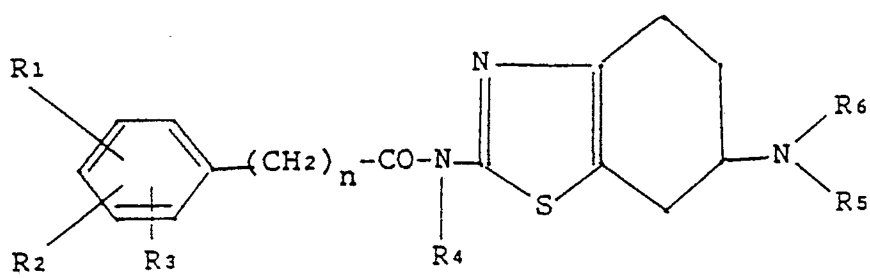

(I),

dans laquelle
n représente 1, 2 ou 3,

R1 représente H, F, Cl, Br, CH3, C2H5, OCH3, OC2H5, OH, CF3,
R2 représente H, Cl, CH3, OCH3, OC2H5, OH,
R3 représente H, NH2,
R4 représente H, CH3, C2H5,
R5 et R6 représentent H, un groupe alkyle en C1-C4, un groupe allyle,
et leurs sels d'addition d'acide.

**2.** Composés selon la revendication 1, dans lesquels
R1 représente H, OCH3, OH, Cl, Br, CH3 ou C2H5,
R2 représente H, OCH3 ou Cl,
R3 représente H ou NH2,
R4 représente H ou CH3,
R5' représente H, un groupe alkyle en C1-C3 ou un groupe allyle,
R6 représente H, un groupe alkyle en C1-C3 ou un groupe allyle et
n a la signification ci-dessus.

**3.** Composés selon la revendication 1, dans lesquels R1 représente OCH3 ou OH,
R2, R3 et R4 représentent H,
R5 et R6 représentent H ou un groupe alkyle en C1-C3 et n a la signification ci-dessus.

**4.** Composés selon l'une des revendications précédentes, dans lesquels n signifie 2 ou 3.

**5.** Composés selon la revendication 1, 2 ou 3, dans lesquels n signifie 2.

**6.** 2-(4-méthoxyphénylpropionyl)amino-6-n-propylamino-4,5,6,7-tétrahydrobenzothiazole sous forme de racémate ou de l'énantiomère actif, dans chaque cas sous forme de base libre ou des sels d'addition d'acides.

**7.** Médicament, caractérisé en ce qu'il contient un composé selon l'une des revendications 1 à 6, en plus des adjuvants et/ou véhicules habituels.

**8.** Utilisation de composés selon l'une des revendications 1 à 6 pour la préparation de médicaments pour le traitement de la maladie de Parkinson ou du parkinsonisme, pour le traitement de la schizophrénie et pour l'inhibition de la prolactine.

**9.** Procédé de préparation des composés selon les revendications 1 à 6 d'après des méthodes connues en soi, caractérisé en ce que l'on fait réagir

a) un composé de formule

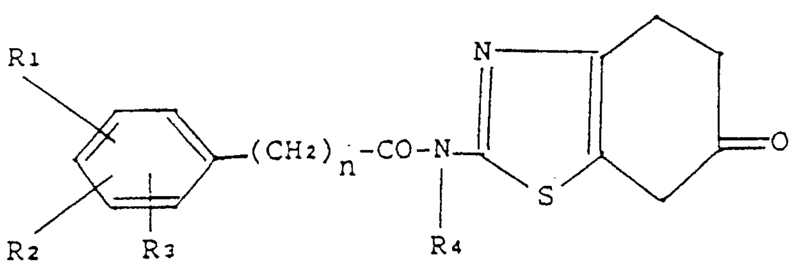

(III)

avec un composé de formule

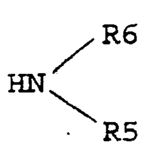

(IV)

dans les conditions d'une amination réductrice
ou en ce que
b) pour préparer un composé de formule I comportant un groupe OH phénolique l'on soumet un composé correspondant comportant un groupe éther à une coupure de l'éther
ou en ce que
c) pour préparer des composés de formule I dans lesquels les deux restes R5 et R6 ne représentent pas des atomes d'hydrogène, on fait réagir un composé de formule I dans laquelle R6 est un atome d'hydrogène avec un composé

x - R'6 (V)

qui convient à l'introduction du reste R'6 (R'6 est identique à R6 sauf en ce qui concerne l'hydrogène), R'6 ayant la signification donnée ci-dessus et x représentant un groupe éliminable par introduction du groupe R'6 dans le groupe amine,
et en ce que l'on sépare si on le souhaite les racémates obtenus en les énantiomères et/ou l'on convertit les bases obtenues en sels d'addition d'acides, les sels d'addition d'acide obtenus en les bases libres ou en des sels d'autres acides.